# EUROPÄISCHE PATENTSCHRIFT

(11) **EP 2 135 567 B1**
(45) Veröffentlichungstag und Bekanntmachung des Hinweises auf die Patenterteilung: **06.08.2014**
(21) Anmeldenummer: 08158712.3
(22) Anmeldetag: 20.06.2008
(51) Int. Cl.: A61B 17/16, H02K 5/128, H02K 21/14

(54) **Chirurgiemotor, chirurgische Maschine und Verfahren zum Herstellen eines Chirurgiemotors**
Surgical motor, surgical machine and method for producing a surgical motor
Moteur chirurgical, machine chirurgicale et procédé de fabrication d'un moteur chirurgical

(43) Veröffentlichungstag der Anmeldung: 23.12.2009
(60) Teilanmeldung: 12176259.5
(73) Patentinhaber: Aesculap AG, 78532 Tuttlingen (DE)
(72) Erfinder: Kahler, Thomas, 78606 Seitingen-Oberflacht (DE); Hoegerle, Roland, 78532 Tuttlingen (DE)
(74) Vertreter: Hoeger, Stellrecht & Partner Patentanwälte

(56) Entgegenhaltungen:
- WO-A-2006/111173
- DE-U1- 20 202 724
- US-A- 5 093 593

## Beschreibung

Die vorliegende Erfindung betrifft einen elektrischen Chirurgiemotor mit einem Stator und einem im Stator rotierbar gelagerten Rotor, wobei der Stator mindestens zwei Statorwicklungen umfasst, wobei der Stator eine Schutzhülse umfasst und wobei die mindestens zwei Statorwicklungen die Schutzhülse umgeben.

Ferner betrifft die vorliegende Erfindung eine chirurgische Maschine mit einem Antrieb zum Bewegen eines Bearbeitungswerkzeugs, welches direkt oder indirekt mit dem Antrieb koppelbar ist, wobei der Antrieb ein elektrischer Chirurgiemotor der eingangs beschriebenen Art ist.

Des Weiteren betrifft die vorliegende Erfindung ein Verfahren zum Herstellen eines elektrischen Chirurgiemotors mit einem Stator und einem im Stator rotierbar gelagerten Rotor, wobei der Stator mindestens zwei Statorwicklungen umfasst.

Eine chirurgische Maschine mit einem elektrischen Chirurgiemotor der eingangs beschriebenen Art ist beispielsweise aus der DE 202 02 724 U1 bekannt. Um möglichst große Leistungen bei kleiner Bauform der elektrischen Chirurgiemotoren erreichen zu können, die insbesondere in Form elektronisch kommutierter Gleichstrommotoren ausgebildet sein können, werden die mindestens zwei Statorwicklungen, üblicherweise sind es drei, freitragend gewickelt werden, es ist also kein Spulenträger vorgesehen, auf den die mindestens zwei Statorwicklungen gewickelt werden. Zur Erhöhung der Stabilität der Statorwicklungen werden diese nach dem Wickeln vergossen. Weitere Chirurgiemotoren und chirurgische Maschinen der eingangs beschriebenen Art sind aus der WO 2006/111173 A1 sowie aus der US 5,093,593 bekannt. Chirurgische Maschinen und damit auch elektrische Chirurgiemotoren müssen je nach Einsatzzweck dampfsterilisierbar sein. Dies bedeutet, dass sie Heißdampf mit Überdruck und Temperaturen über 120°C ausgesetzt werden. Dies kann zur Folge haben, dass durch die Dampfsterilisation sowie die zusätzliche, optionale Aufbereitung entsprechend der Empfehlung des Robert-Koch-Instituts (RKI) mit Medien mit einem pH-Wert von 12 bis 14, ein durch Vergießen entstandener Wicklungsverbund der mindestens zwei Statorwicklungen geschwächt oder zerstört werden kann. Folge hiervon kann sein, dass sich aufgrund von Verformungskräften innerhalb der geformten Statorwicklungen ein Innendurchmesser der Statorwicklungen ändern kann, insbesondere kann er kleiner werden. Im schlimmsten Fall kann dann der Rotor des Chirurgiemotors an den Statorwicklungen streifen und diese weiter zerstören. Daraus kann ein weiterer Nachteil resultieren, nämlich der, dass eine erforderliche elektrische Isolation der Maschine zum Anwender beziehungsweise Patienten verloren gehen kann, da durch die Abnutzung ein direkter galvanischer Kontakt zwischen den Statorwicklungen und dem Rotor des Motors hergestellt werden kann.

Um die genannten Probleme möglichst zu vermeiden, müssen große Luftspalte zwischen Stator und Rotor vorgesehen werden. Zur Konstruktion eines leistungsstarken Chirurgiemotors ist es jedoch wünschenswert, den Luftspalt zwischen Stator und Rotor möglichst klein auszubilden. Damit ist er jedoch konstruktionsbedingt auch anfällig gegen Verschmutzungspartikel, die beim Einsatz oder während der Aufbereitung und Sterilisation in den Luftspalt eindringen können. Diese Partikel können sich im Luftspalt verklemmen und führen bei entsprechenden Drehzahlen, die bei Chirurgiemotoren ohne Weiteres im Bereich von 100.000 bis 200.000 Umdrehungen pro Minute liegen können, schnell zu einer Erhitzung und Zerstörung der zur Stabilisierung der Statorwicklungen verwendeten Materialien.

Es ist daher Aufgabe der vorliegenden Erfindung, einen Chirurgiemotor, eine chirurgische Maschine sowie ein Verfahren der eingangs beschriebenen Art so zu verbessern, dass der Chirurgiemotor bei minimaler Störanfälligkeit möglichst leistungsstark ist.

Diese Aufgabe wird bei einem elektrischen Chirurgiemotor sowie einer chirurgischen Maschine mit den Merkmalen des Anspruchs 1 bzw. 15 gelöst.

Die Schutzhülse hat insbesondere den Vorteil, dass sie die Statorwicklungen des Chirurgiemotors vor äußeren Einflüssen schützt. Insbesondere kann der Chirurgiemotor so ausgebildet sein, dass die, vorzugsweise ein separates Bauteil bildende Schutzhülse verhindert, dass beispielsweise Heißdampf und/oder Verschmutzungspartikel mit den mindestens zwei Statorwicklungen und/oder einer Einbett- oder Vergussmasse, in die die Statorwicklungen eingebettet sind, in Kontakt treten kann. Die Schutzhülse definiert selbst einen Innendurchmesser, der durch äußere Einflüsse praktisch nicht veränderbar ist. Dies hat jedoch den Vorteil, dass ein Luftspalt zwischen dem Stator und dem Rotor minimal klein ausgebildet werden kann. Je kleiner der Luftspalt zwischen Rotor und Stator ist, umso leistungsstärker ist der Chirurgiemotor. Der erfindungsgemäß vorgeschlagene Chirurgiemotor eignet sich somit hervorragend, um dampfsterilisiert zu werden, beispielsweise auch im Einbauzustand wie bei einer in der DE 202 02 724 U1 beschriebenen chirurgischen Maschine. Um Kurzschlüsse zu verhindern, ist es vorteilhaft, dass die Schutzhülse aus einem elektrisch isolierenden Material hergestellt ist. Besonders stabil kann die Schutzhülse ausgebildet und für eine Heißdampfsterilisation sowie Behandlung mit Säuren, insbesondere mit pH-Werten größer als 12, geeignet werden, da sie aus einer Keramik hergestellt ist.

Um zu verhindern, dass sich die Schutzhülse bei einer Reinigung und Aufbereitung des Chirurgiemotors in unerwünschter Weise verformen kann, ist es günstig, wenn sie aus einem dampfsterilisierbaren Material hergestellt ist. So kann insbesondere sichergestellt werden, dass ein Innendurchmesser der Schutzhülse und damit auch der mindestens zwei Statorwicklungen sich auch nach vielen Aufbereitungszyklen nicht ändern kann. Vorzugsweise ist das dampfsterilisierbare Material ein Material, das sich bei einer Heißdampfsterilisation üblicherweise herrschenden Temperaturen nicht verändert, insbesondere nicht verformt.

Vorteilhaft ist es, wenn zwischen dem Stator und dem Rotor ein Luftspalt ausgebildet ist und wenn die Schutzhülse direkt an den Luftspalt angrenzt. Die Schutzhülse kann so verhindern, dass die mindestens zwei Statorwicklungen in den Luftspalt eindringen und mit dem Rotor in Kontakt kommen können, beispielsweise infolge einer Verformung der Statorwicklungen oder von diese umgebendem Einbettungsmaterial.

Besonders leistungsstarke Chirurgiemotoren können ausgebildet werden, wenn der Luftspalt höchstens 1 mm breit ist. Vorzugsweise ist er jedoch weniger als 0,5 mm breit. Besonders günstig ist es, wenn er höchstens 0,1 mm breit ist.

Vorteilhafterweise weist die Schutzhülse eine Dicke von maximal 0,5 mm auf. Dies hat den Vorteil, dass eine optimale magnetische Ankopplung zwischen Rotor und Stator möglich ist, also eine nur geringe Änderung der von Rotor und Stator erzeugten Magnetfelder eintreten kann. Insbesondere ist es günstig, wenn die Schutzhülse eine Dicke von maximal 0,3 mm aufweist, besser noch eine Dicke von maximal 0,1 mm.

Besonders gute Stabilitätseigenschaften weisen Keramiken in Form von Oxidkeramiken auf.

Vorzugsweise handelt es sich bei der Oxidkeramik um Aluminiumoxid (Al₂O₃), Zirkoniumdioxid (ZrO₂), Titanoxid (TiO₂), Magnesiumoxid (MgO), Zinkoxid (ZnO), Aluminiumtitanat (Al₂O₃ + TiO₂) und/oder um Bariumtitanat (BaO + TiO₂) beziehungsweise die Oxidkeramik enthält eines oder mehrere der genannten Materialien.

Ein besonders einfacher Aufbau des Chirurgiemotors kann erreicht werden, indem der Rotor einen stabförmigen, vorzugsweise zylindrischen Permanentmagneten umfasst.

Um eine Beschädigung des Permanentmagneten zu verhindern, ist er vorzugsweise von einer hülsenförmigen Rotorarmierung umgeben. Die Rotorarmierung ist günstigerweise aus einem elektrisch nicht leitenden Material hergestellt.

Vorteilhafterweise ist der Luftspalt zwischen der Schutzhülse und der Rotorarmierung ausgebildet. Durch die Schutzhülse und die Rotorarmierung kann der Luftspalt zwischen Rotor und Stator somit optimal geschützt und zudem verhindert werden, dass Teile des Stators beziehungsweise Rotors in ihn eindringen können. Die Rotorarmierung dient insbesondere dazu, zu verhindern, dass sich ein Durchmesser des Permanentmagneten, aus welchen Gründen auch immer, vergrößern kann.

Vorzugsweise ist die Rotorarmierung aus einem magnetisch leitfähigen Material hergestellt. So können die vom Stator beziehungsweise Rotor erzeugten Magnetfelder die Rotorarmierung optimal durchdringen.

Um einen besonders hohen Stromfluss durch die Statorwicklungen erreichen zu können, ist es günstig, wenn die mindestens zwei Statorwicklungen aus Kupfer hergestellt sind.

Damit die Stabilität der Statorwicklung weiter erhöht sowie optional ein Verformen derselben verhindert werden kann, ist es vorteilhaft, wenn sie in einer Einbett- oder Vergussmasse eingebettet sind.

Um einen möglichst kompakten Aufbau des Chirurgiemotors zu erreichen, ist es günstig, wenn die mindestens zwei Statorwicklungen und die Einbett- oder Vergussmasse gemeinsam eine Wicklungsschicht bilden oder definieren. Die Wicklungsschicht kann insbesondere direkt an die Schutzhülse angrenzen. Beispielsweise können die mindestens zwei freitragend gewickelten Statorwicklungen nach dem Wickeln und Einführen einer als separates Bauteil ausgebildeten Schutzhülse mit der Einbett- oder Vergussmasse vergossen werden. Auf diese Weise können die mindestens zwei Statorwicklungen auch direkt mit der Schutzhülse verbunden werden, um beispielsweise eine unerwünschte Relativbewegung der Schutzhülse und der Wicklungsschicht zu vermeiden.

Um Kurzschlüsse zu verhindern, ist es günstig, wenn die Einbett- oder Vergussmasse elektrisch isolierend ist.

Vorteilhafterweise ist die Einbett- oder Vergussmasse magnetisch leitend. Dies ermöglicht eine optimale Durchdringung durch die vom Rotor und Stator erzeugten Magnetfelder.

Besonders einfach in der Herstellung wird der Chirurgiemotor, wenn die Einbett- oder Vergussmasse aus dem gleichen Material hergestellt ist, wie die Schutzhülse. Ferner hat dies den Vorteil, dass Wärmeausdehnungskoeffizienten der Schutzhülse und der Einbett- oder Vergussmasse gleich sind, was insbesondere bei großen Temperaturschwankungen, die beispielsweise bei einer Heißdampfsterilisation des Chirurgiemotors auftreten können, vorteilhaft ist.

Günstigerweise ist die Einbett- oder Vergussmasse dotiert. Insbesondere ist sie dotiert, um sie, wenn sie von Haus aus nicht magnetisch leitfähig ist, magnetisch leitfähig zu machen.

Vorzugsweise ist die Einbett- oder Vergussmasse schichtweise dotiert. Insbesondere können mehrere abwechselnd dotierte und undotierte Schichten ausgebildet werden, die die mindestens zwei Statorwicklungen umgeben.

Um eine ausreichende magnetische Leitfähigkeit beziehungsweise magnetische Permeabilität µ erreichen zu können, ist es vorteilhaft, wenn die Einbett- oder Vergussmasse mit magnetischen und/oder magnetisierbaren Partikeln dotiert ist. Diese können insbesondere kleine inselartige Einschlüsse bilden, die durch die Einbett- oder Vergussmasse voneinander getrennt sind.

Hervorragend zum Dotieren der Einbett- oder Vergussmasse, um diese magnetisch leitfähig zu machen, eignen sich magnetische und/oder magnetisierbare Partikel, welche Bornitrit, Titan-Bornitrit, Eisen und/oder Nickel enthalten beziehungsweise ganz aus einem dieser Materialien bestehen.

Insbesondere dann, wenn es sich bei den Materialen zum Dotieren der Einbett- oder Vergussmasse um elektrisch leitfähige Materialien handelt, ist es günstig, wenn die magnetischen und/oder magnetisierbaren Partikel gegeneinander elektrisch isoliert sind. So können insbesondere unerwünschte Kurzschlüsse in der Wicklungsschicht vermieden werden.

Um die mindestens zwei Statorwicklungen beziehungsweise die Wicklungsschicht zusätzlich zu schützen, ist es günstig, wenn die mindestens zwei Statorwicklungen zwischen der Schutzhülse und einer elektrisch isolierenden Isolationsschicht angeordnet sind.

Der Aufbau des Chirurgiemotors wird besonders einfach, wenn die Schutzhülse und die Isolationsschicht aus dem gleichen Material hergestellt sind. Dies hat ferner den Vorteil, dass wiederum auch die Ausdehnungskoeffizienten der unterschiedlichen Schichten gleich sind, was insbesondere bei großen Temperaturschwankungen, wie sie beim Heißdampfsterilisieren auftreten können, eine unerwünschte Zerstörung des Chirurgiemotors verhindern kann.

Um den Aufbau des Chirurgiemotors möglichst kompakt halten zu können, ist es vorteilhaft, wenn die Wicklungsschicht direkt zwischen der Schutzhülse und der Isolationsschicht angeordnet ist.

Um die Verlustleistung des Chirurgiemotors möglichst klein zu halten, ist es günstig, wenn die mindestens zwei Statorwicklungen von einer magnetischen Rückschlusseinrichtung umgeben sind. Diese hält die erzeugten Magnetfelder im Wesentlichen im Innern des Chirurgiemotors.

Damit möglichst keine Wirbelströme induziert werden können, die für den Rotor als Bremse wirken können, umfasst die magnetische Rückschlusseinrichtung günstigerweise mindestens eine Metallschicht.

Vorzugsweise sind mehrere Metallschichten vorgesehen, beispielsweise drei, vier, fünf, sechs oder sieben, durch die Wirbelströme verhindert werden beziehungsweise minimiert werden können. Ferner eignet sich eine derart aufgebaute Rückschlusseinrichtung hervorragend, um magnetische Feldlinien der vom Stator und Rotor erzeugten Magnetfelder kurzzuschließen.

Besonders wirkungsvoll wird die Rückschlusseinrichtung, wenn die mindestens eine Metallschicht aus Eisen hergestellt ist oder Eisen enthält. Mit anderen Worten ist die Metallschicht vorzugsweise aus einem magnetischen oder zumindest magnetisierbaren Material hergestellt, und zwar entweder ganz oder zumindest teilweise.

Vorteilhafterweise ist die magnetische Rückschlusseinrichtung im Wesentlichen rohrförmig ausgebildet. Dadurch ist es möglich, den gesamten Stator zu umgeben und die durch Rotor und Stator erzeugten Magnetfelder im Wesentlichen im Innern des Chirurgiemotors zu halten.

Besonders kompakt aufbauen lässt sich der Chirurgiemotor, wenn die magnetische Rückschlusseinrichtung direkt an die Isolationsschicht angrenzt.

Ein besonders guter Schutz des Chirurgiemotors lässt sich erreichen, wenn ein den Stator umgebendes Stator- oder Motorgehäuse vorgesehen ist.

Besonders einfach wird der Aufbau des Chirurgiemotors, wenn der Stator und/oder der Rotor rotationssymmetrisch oder im Wesentlichen rotationssymmetrisch ausgebildet sind. Insbesondere ist eine solche Symmetrie durch einen schichtförmigen Aufbau des Rotors und auch des Stators zu erzielen.

Vorzugsweise umfasst der Chirurgiemotor drei Statorwicklungen, diese können insbesondere sternförmig miteinander verschaltet sein.

Um die Statorwicklungen auf einfache Weise mit einer elektrischen Energieversorgung verbinden zu können, beispielsweise mit einer Batterie oder einer netzgebundenen Strom-/Spannungsversorgungseinheit, ist es vorteilhaft, wenn der Chirurgiemotor eine elektrische Anschlusseinrichtung umfasst mit mindestens zwei Anschlusskontakten, welche mit den mindestens zwei Statorwicklungen elektrisch leitend verbunden sind. Beispielsweise können die Anschlusskontakte in Form von Steckkontakten ausgebildet sein, welche mit korrespondierenden Anschlusselementen an einem Gehäuse der chirurgischen Maschine in Eingriff gebracht werden können, um den Motor zum Beispiel mit einer Steuer- und/oder Regelungseinrichtung, die im Gehäuse angeordnet ist, zu verbinden.

Um die Statorwicklungen des Chirurgiemotors separat ansteuern zu können, ist es günstig, wenn die Zahl der Anschlusskontakte der Zahl der Statorwicklungen entspricht. Vorzugsweise sind die Anschlusskontakte mit jeweils zwei direkt miteinander verbundenen Statorwicklungen elektrisch leitend verbunden.

Die eingangs gestellte Aufgabe wird ferner bei einem Verfahren zum Herstellen eines elektrischen Chirurgiemotors mit den Merkmalen des Anspruchs 16 gelöst.

Auf diese Weise können die Statorwicklungen zunächst in gewünschter Weise und Form gewickelt werden. Erst nach deren Wicklung wird die Schutzhülse eingeführt oder eingesetzt, die eine unerwünschte Ausdehnung der mindestens zwei Statorwicklungen beziehungsweise von Teilen derselben in Richtung auf eine vom Stator definierte Längsachse hin verhindern helfen. Um Kurzschlüsse zu verhindern, ist es vorteilhaft, dass die Schutzhülse aus einem elektrisch isolierenden Material hergestellt ist. Besonders stabil kann die Schutzhülse ausgebildet und für eine Heißdampfsterilisation sowie Behandlung mit Säuren, insbesondere mit pH-Werten größer als 12, geeignet werden, da sie Schutzhülse aus einer Keramik hergestellt ist.

Des Weiteren ist es günstig, wenn die mindestens zwei Statorwicklungen nach dem Einführen der Schutzhülse mit einer Einbett- oder Vergussmasse vergossen werden zum Ausbilden einer die mindestens zwei Statorwicklungen enthaltenden Wicklungsschicht. Dies erhöht zusätzlich die Stabilität des Stators. Insbesondere kann so die Schutzhülse auch als Träger oder zur Formgebung der Wicklungsschicht dienen, denn die Schutzhülse verhindert so auch, dass in einen Luftspalt zwischen Stator und Rotor vorstehende und im ungünstigsten Fall den Rotor berührende Vorsprünge der Einbett- oder Vergussmasse ausgebildet werden können.

Auf einfache Weise lässt sich die Stabilität des Stators erhöhen, wenn die mindestens zwei Statorwicklungen und die Schutzhülse durch das Vergießen mit der Einbett- oder Vergussmasse miteinander verbunden werden. Als Einbett-oder Vergussmasse eignen sich insbesondere Tränkharze, verbackbare Backlacke, spritzbare Thermoplaste oder spritzbare Gießharze, beispielsweise Epoxydharze.

Insbesondere ist es vorteilhaft, wenn das Verfahren zum Herstellen eines der oben beschriebenen Chirurgiemotoren verwendet wird.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht einer chirurgischen Maschine mit einem elektrischen Chirurgiemotor;
- Figur 2:: eine teilweise geschnittene perspektivische Ansicht eines elektrischen Chirurgiemotors;
- Figur 3:: eine Schnittansicht längs Linie 3-3 in Figur 2;
- Figur 4:: eine schematische Schnittansicht durch die Einbett- oder Vergussmasse der Wicklungsschicht; und
- Figur 5:: eine schematische vergrößerte Schnittansicht durch eine dotierte Schicht der Wicklungsschicht.

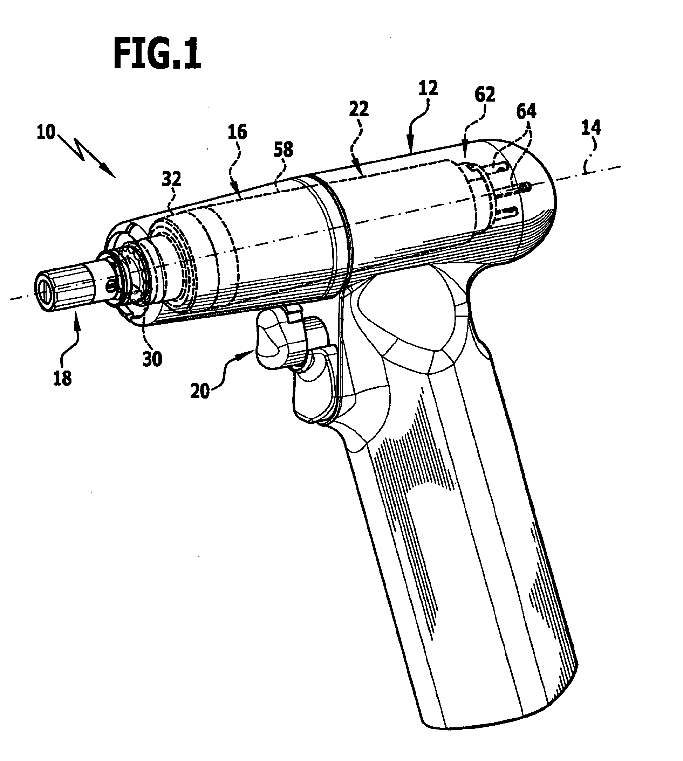

In Figur 1 ist eine insgesamt mit dem Bezugszeichen 10 versehene chirurgische Maschine dargestellt, welche ein Gehäuse 12 mit einer Aufnahme für eine nicht dargestellte Batterie sowie einen im Gehäuse 12 angeordneten und eine Längsachse 14 definierenden elektrischen Chirurgiemotor 16 aufweist, beispielsweise in Form eines elektronisch kommutierten Gleichstrommotors.

Abtriebsseitig ist der Chirurgiemotor 16 mit einer aus dem Gehäuse 12 herausgeführten Kupplungseinrichtung 18 verbunden, mittels derer die Maschine 10 mit einem nicht dargestellten Bearbeitungswerkzeug, beispielsweise einem Bohrer oder Sägeblatt, verbunden werden kann zum Bohren oder Sägen. Am Gehäuse 12 ist ferner ein beweglich gelagertes Betätigungselement 20 angeordnet, welches mit einer nicht näher dargestellten Steuer- und/oder Regelungseinheit der Maschine 10 verbunden ist. Das Betätigungselement 20 dient insbesondere als Eingabeelement zum Vorgeben einer gewünschten Drehzahl und/oder Drehrichtung des Chirurgiemotors 16. Man kann das Betätigungselement 20 umgangssprachlich auch als "Gasdrücker" oder als Drehzahlvorgabeglied bezeichnen.

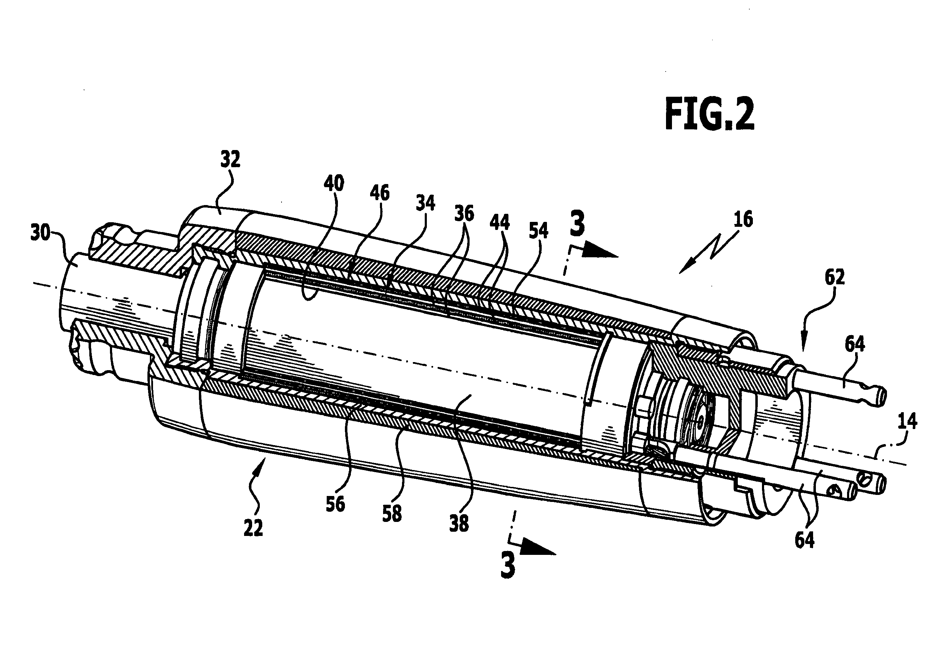

Der Chirurgiemotor 16 umfasst, wie in Figur 2 dargestellt, einen Stator 22 sowie einen in diesem um die Längsachse 14 rotierbar gelagerten Rotor 24. Der Rotor 24 umfasst einen langgestreckten, zylindrischen Dauer- oder Permanentmagnet 26, der spaltfrei von einer dünnwandigen, hülsenförmigen Rotorarmierung 28 umgeben ist. Der Rotor 24 insgesamt ist abtriebsseitig in Form eines lagerwellenartigen Abschnitts 30 ausgebildet, welcher in einem hülsenförmigen Abschnitt eines Statorgehäuses 32 geführt ist. Für eine optimale, möglichst reibungsfreie Lagerung des Rotors 24 im Stator 22 sind optional geeignete Gleit- oder Kugellager im Bereich der Endabschnitte des Permanentmagneten 26 konzentrisch zur Längsachse 14 angeordnet.

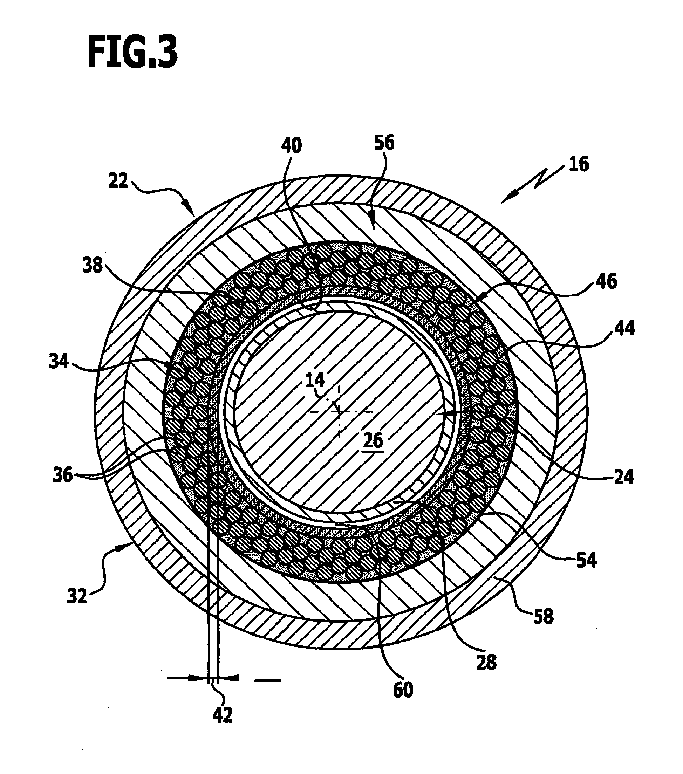

Der Stator 22 selbst ist im Wesentlichen in Form einer langgestreckten Hülse ausgebildet, zumindest im Bereich des Rotors 24. Er ist schichtförmig ausgebildet beziehungsweise aufgebaut, wie insbesondere in Figur 3 gut zu erkennen. Zur Herstellung des Stators 22 werden zunächst drei, Motorwicklungen bildende Statorwicklungen 34 freitragend in bekannter Weise aus elektrisch leitfähigen Drähten 36, vorzugsweise aus Kupfer, gewickelt, so dass insgesamt durch die Statorwicklungen 34 eine Drahthülse ausgebildet wird.

In einem nächsten Schritt wird eine Schutzhülse 38 mit einer Dicke 42 in die von den Statorwicklungen 34 definierte hohlzylindrische Ausnehmung 40 eingeschoben. Ein Außendurchmesser der Schutzhülse 38 entspricht im Wesentlichen einem Innendurchmesser der Statorwicklungshülse. Die Dicke 42 der Schutzhülse 38 beträgt maximal 0,5 mm, vorzugsweise ist sie jedoch kleiner, insbesondere 0,4, 0,3 oder 0,2 mm. Die Schutzhülse ist aus einer magnetisch nichtleitenden Keramik, vorzugsweise einer Oxidkeramik, hergestellt, welche Aluminiumoxid, Zirkoniumdioxid, Titanoxid, Magnesiumoxid, Zinkoxid, Aluminiumtitanat und/oder Bariumtitanat enthält oder ganz aus einem der genannten Materialien hergestellt ist. Die Schutzhülse 38 ist elektrisch isolierend, optional kann sie durch entsprechende Dotierung mit magnetischen und/oder magnetisierbaren Partikeln magnetisch leitfähig gemacht werden.

Die Rotorarmierung 28 ist vorzugsweise aus einem elektrisch nicht leitenden, also isolierenden Material hergestellt, welches jedoch vorzugsweise magnetisch leitfähig ist, so dass es von den vom Rotor 24 und Stator 22 erzeugten Magnetfeldern durchdrungen werden kann.

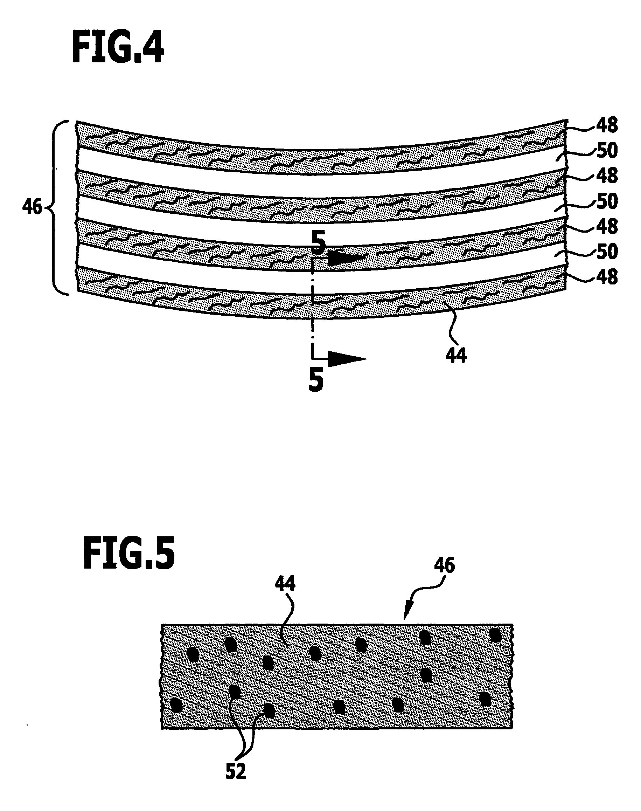

In einem nächsten Herstellungsschritt werden die Statorwicklungen 34 in eine Einbett- oder Vergussmasse 44 eingebettet, beispielsweise durch Vergießen. Die Vergussmasse ist vorzugsweise elektrisch nichtleitend, jedoch magnetisch leitend. Insbesondere kann die Vergussmasse ein Tränkharz, ein verbackbarer Backlack, ein umspritzbarer Thermoplast oder ein gießfähiges Gießharz, beispielsweise ein Epoxydharz, sein, oder aber aus dem gleichen Material wie die Schutzhülse 38 bestehen. Um das Einbettmaterial beziehungsweise die Vergussmasse 44, welche ursprünglich nicht magnetisch leitfähig ist, magnetisch leitfähig zu machen, wird sie vorzugsweise schichtweise dotiert. In Figur 4 ist beispielhaft der Aufbau einer durch die in der Vergussmasse 44 eingebetteten Statorwicklungen 34 ausgebildete Wicklungsschicht 46 im Schnitt dargestellt, welche vier dotierte Schichten 48 umfasst, die durch drei undotierte Schichten 50 voneinander getrennt sind. Die Dotierung erfolgt durch Einbringen von magnetischen oder magnetisierbaren Partikeln 52, bei denen es sich beispielsweise um Bornitrit, Titan-Bornitrit, Eisen und/oder Nickel enthaltende Partikel handeln kann oder um Partikel, die ganz aus den genannten Materialien bestehen. Denkbar sind auch alle anderen Materialien, die magnetisierbar sind. Die Partikel 52 sind elektrisch gegeneinander isoliert durch die aus einem elektrisch isolierenden Material hergestellte Vergussmasse 44. Bei der Vergussmasse 44 kann es sich insbesondere um das gleiche Material handeln, wie das, aus dem die Schutzhülse 38 hergestellt ist.

Die Wicklungsschicht 46 ist, von der Längsachse 14 weg weisend, von einer Isolationsschicht 54 umgeben, die aus einem elektrisch isolierenden Material hergestellt ist. Es kann sich insbesondere um das gleiche Material handeln wie das, aus dem die Schutzhülse 38 hergestellt ist.

Die Isolationsschicht 54 wiederum wird umgeben von einer magnetischen Rückschlusseinrichtung 56, welche, wie alle Schichten des Stators 22, hülsen-oder rohrförmig ausgebildet ist. Die Rückschlusseinrichtung 56 selbst kann mehrere hülsenförmig ausgebildete Metallschichten umfassen, welche die Längsachse 14 konzentrisch umgeben. Vorzugsweise sind sie aus Eisen oder einem Eisen enthaltenden Metall hergestellt. Denkbar sind aber auch alle anderen, bekannten magnetisierbaren Materialien. Die Metallschichten der Rückschlusseinrichtung 56 sind vorzugsweise durch Isolationsschichten elektrisch gegeneinander isoliert. Außen wird die Rückschlusseinrichtung 56 von einem hülsenförmigen Abschnitt 58 des Motor- oder Statorgehäuses 32 umgeben.

Um eine reibungsfreie Rotation des Rotors 24 im Stator 22 zu gewährleisten, ist zwischen der Rotorarmierung 28 und der Schutzhülse 38 ein schmaler Luftspalt 60 ausgebildet, welcher maximal 1 mm breit ist, vorzugsweise jedoch deutlich schmaler, insbesondere kleiner als 0,5 mm oder sogar, wenn dies fertigungstechnisch möglich ist, kleiner als 0,1 mm.

Die Isolationsschicht 54, die Schutzhülse 38 sowie die Vergussmasse 44 können alle aus dem gleichen Material gebildet sein. Die Isolationsschicht kann insbesondere auch durch eine eine dotierte Schicht 48 der Wicklungsschicht 46 umgebende undotierte Schicht 50 gebildet sein.

Der beschriebene Aufbau des Stators ermöglicht es, dass die Rückschlusseinrichtung 56 aus Einzelblechen mit einer geringen Dicke ausgeführt werden oder sogar ganz entfallen kann.

Der Chirurgiemotor 16 ist ferner mit einer Anschlusseinrichtung 62 umfassend drei stiftförmige, parallel zueinander ausgerichtete und parallel zur Längsachse 14 verlaufende Anschlusskontakte 64 ausgestattet. Die Anschlusskontakte 64 können beispielsweise mit nicht dargestellten korrespondierenden Kontaktelementen im Gehäuse 12 verbunden werden, um eine elektrisch leitende Verbindung zu einer Steuer- oder Regelungseinrichtung der Maschine 10 sowie deren Energieversorgung herzustellen.

Die Schutzhülse 38 weist eine hohe Druckfestigkeit auf, so dass innerhalb der Wicklungsschicht 46 auftretende Verformungskräfte gut aufgenommen werden können. Dies sichert einen vorgegebenen Innendurchmesser der Schutzhülse 38 und damit eine Breite des Luftspalts 60, so dass Kontakte zwischen Rotor 24 und Stator 22 dauerhaft vermieden werden können.

Die aus einer Oxidkeramik hergestellte Schutzhülse 38 bietet einen hohen Widerstand gegenüber Abrieb und Verschleiß, die insbesondere beim Kontaktieren von eingedrungenen Partikeln bei entsprechend hohen Drehzahlen an den exponierten Flächen auftreten können.

Die Schutzhülse 38 ermöglicht es zudem, die Statorwicklungen 34 abzustützen und gestattet es so, Chirurgiemotoren 16 besonders kleiner Baugröße, das heißt besonders kleiner Durchmesser, zu fertigen, insbesondere sind Außendurchmesser des Statorgehäuses 32 von weniger als 15 mm möglich.

Des Weiteren bietet die Schutzhülse 38 den Statorwicklungen 34 sowie der Vergussmasse 44 einen optimalen Schutz vor widrigen Umgebungseinflüssen, wie sie insbesondere bei einer Heißdampfsterilisation herrschen, das heißt thermische, mechanische und chemische Einflüsse, die insbesondere zu einer Verformung der Wicklungsschicht 46 führen können. Des Weiteren ist ein Verschleißverhalten des Chirurgiemotors 16 gegenüber eingedrungenen Partikeln verbessert, so dass insgesamt eine deutliche höhere Standzeit des Chirurgiemotors 16 erreicht werden kann.

Durch den aufgrund der Schutzhülse 38 sehr schmal ausbildbaren Luftspalt 60 kann die Motorleistung bei gleichen Außenabmessungen des Chirurgiemotors 16 deutlich verbessert werden, da Leistungsverluste durch große Luftspalte vermieden werden.

## Patentansprüche

1. Elektrischer Chirurgiemotor (16) mit einem Stator (22) und einem im Stator (22) rotierbar gelagerten Rotor (24), wobei der Stator (22) mindestens zwei Statorwicklungen (34) umfasst, wobei der Stator (22) eine Schutzhülse (38) umfasst und wobei die mindestens zwei Statorwicklungen (34) die Schutzhülse (38) umgeben, **dadurch gekennzeichnet, dass** die mindestens zwei Statorwicklungen (34) freitragend gewickelt sind und dass die Schutzhülse (38) aus einer Keramik hergestellt ist.

2. Chirurgiemotor nach Anspruch 1, **dadurch gekennzeichnet, dass** die Schutzhülse (38) aus einem dampfsterilisierbaren Material hergestellt ist.

3. Chirurgiemotor nach Anspruch 1 oder 2, **dadurch gekennzeichnet, dass** zwischen dem Stator (22) und dem Rotor (24) ein Luftspalt (60) ausgebildet ist und dass die Schutzhülse (38) direkt an den Luftspalt (60) angrenzt.

4. Chirurgiemotor nach einem der voranstehenden Ansprüche, **dadurch gekennzeichnet, dass** die Schutzhülse (38) eine Dicke von maximal 0,5 mm aufweist.

5. Chirurgiemotor nach einem der voranstehenden Ansprüche, **dadurch gekennzeichnet, dass** die Schutzhülse (38) aus einem elektrisch isolierenden Material hergestellt ist.

6. Chirurgiemotor nach einem der voranstehenden Ansprüche, **dadurch gekennzeichnet, dass** die Keramik eine Oxidkeramik ist.

7. Chirurgiemotor nach Anspruch 6, **dadurch gekennzeichnet, dass** die Oxidkeramik Aluminiumoxid (Al₂O₃), Zirkoniumdioxid (ZrO₂), Titanoxid (TiO₂), Magnesiumoxid (MgO), Zinkoxid (ZnO), Aluminiumtitanat (Al₂O₃ + TiO₂) und/oder Bariumtitanat (BaO + TiO₂) ist und/oder enthält.

8. Chirurgiemotor nach einem der voranstehenden Ansprüche, **dadurch gekennzeichnet, dass** die mindestens zwei Statorwicklungen (34) in einer Einbett- oder Vergussmasse (44) eingebettet sind und dass die mindestens zwei Statorwicklungen (34) und die Einbett- oder Vergussmasse (44) eine Wicklungsschicht (46) bilden.

9. Chirurgiemotor nach Anspruch 8, **dadurch gekennzeichnet, dass** die Einbett- oder Vergussmasse (44) elektrisch isolierend ist.

10. Chirurgiemotor nach Anspruch 8 oder 9, **dadurch gekennzeichnet, dass** die Einbett- oder Vergussmasse (44) magnetisch leitend ist.

11. Chirurgiemotor nach einem der Ansprüche 8 bis 10, **dadurch gekennzeichnet, dass** die Einbett- oder Vergussmasse (44) aus dem gleichen Material hergestellt ist wie die Schutzhülse (38).

12. Chirurgiemotor nach einem der Ansprüche 8 bis 11, **dadurch gekennzeichnet, dass** die Einbett- oder Vergussmasse (44) schichtweise dotiert ist.

13. Chirurgiemotor nach einem der Ansprüche 8 bis 12, **dadurch gekennzeichnet, dass** die Einbett- oder Vergussmasse (44) mit magnetischen und/oder magnetisierbaren Partikeln (52) dotiert ist.

14. Chirurgiemotor nach einem der voranstehenden Ansprüche, **dadurch gekennzeichnet, dass** die mindestens zwei Statorwicklungen (34) zwischen der Schutzhülse (38) und einer elektrisch isolierenden Isolationsschicht (54) angeordnet sind.

15. Chirurgische Maschine (10) mit einem Antrieb (16) zum Bewegen eines Bearbeitungswerkzeugs, welches direkt oder indirekt mit dem Antrieb (16) koppelbar ist, wobei der Antrieb (16) ein elektrischer Chirurgiemotor (16) nach einem der voranstehenden Ansprüche ist.

16. Verfahren zum Herstellen eines elektrischen Chirurgiemotors (16) mit einem Stator (22) und einem im Stator (22) rotierbar gelagerten Rotor (24), wobei der Stator (22) mindestens zwei Statorwicklungen (34) umfasst, **dadurch gekennzeichnet, dass** die Statorwicklungen (34) freitragend oder spulenträgerlos gewickelt werden und dass nach dem Wickeln der mindestens zwei Statorwicklungen (34) eine rohrförmige, aus einer Keramik hergestellte Schutzhülse (38) in die mindestens zwei, in Röhrenform gewickelten Statorwicklungen (34) eingeführt wird.

17. Verfahren nach Anspruch 16, **dadurch gekennzeichnet, dass** die mindestens zwei Statorwicklungen (34) nach dem Einführen der Schutzhülse (38) mit einer Einbett- oder Vergussmasse (44) vergossen werden zum Ausbilden einer die mindestens zwei Statorwicklungen (34) enthaltenden Wicklungsschicht (46).

## Claims

1. Electric surgical motor (16) with a stator (22) and a rotor (24) rotatably mounted in the stator (22), the stator (22) comprising at least two stator windings (34), the stator (22) comprising a protective sleeve (38), and the at least two stator windings (34) surrounding the protective sleeve (38), **characterized in that** the at least two stator windings (34) are wound in a self-supporting manner, and **in that** the protective sleeve (38) is made of a ceramic.

2. Surgical motor in accordance with claim 1, **characterized in that** the protective sleeve (38) is made of a steam-sterilizable material.

3. Surgical motor in accordance with claim 1 or 2, **characterized in that** an air gap (60) is formed between the stator (22) and the rotor (24), and **in that** the protective sleeve (38) borders directly on the air gap (60).

4. Surgical motor in accordance with any one of the preceding claims, **characterized in that** the protective sleeve (38) has a maximum thickness of 0.5 mm.

5. Surgical motor in accordance with any one of the preceding claims, **characterized in that** the protective sleeve (38) is made of an electrically insulating material.

6. Surgical motor in accordance with any one of the preceding claims, **characterized in that** the ceramic is an oxide ceramic.

7. Surgical motor in accordance with claim 6, **characterized in that** the oxide ceramic is and/or contains aluminium oxide (Al₂O₃), zirconium dioxide (ZrO₂), titanium oxide (TiO₂), magnesium oxide (MgO), zinc oxide (ZnO), aluminium titanate (Al₂O₃ + TiO₂) and/or barium titanate (BaO + TiO₂).

8. Surgical motor in accordance with any one of the preceding claims, **characterized in that** the at least two stator windings (34) are embedded in an embedding or casting compound (44), and **in that** the at least two stator windings (34) and the embedding or casting compound (44) form a winding layer (46).

9. Surgical motor in accordance with claim 8, **characterized in that** the embedding or casting compound (44) is electrically insulating.

10. Surgical motor in accordance with claim 8 or 9, **characterized in that** the embedding or casting compound (44) is magnetically conductive.

11. Surgical motor in accordance with any one of claims 8 to 10, **characterized in that** the embedding or casting compound (44) is made of the same material as the protective sleeve (38).

12. Surgical motor in accordance with any one of claims 8 to 11, **characterized in that** the embedding or casting compound (44) is doped in layers.

13. Surgical motor in accordance with any one of claims 8 to 12, **characterized in that** the embedding or casting compound (44) is doped with magnetic and/or magnetizable particles (52).

14. Surgical motor in accordance with any one of the preceding claims, **characterized in that** the at least two stator windings (34) are arranged between the protective sleeve (38) and an electrically insulating insulation layer (54).

15. Surgical machine (10) with a drive (16) for moving a machining tool which can be directly or indirectly coupled to the drive (16), the drive (16) being an electric surgical motor (16) in accordance with any one of the preceding claims.

16. Method for producing an electric surgical motor (16) with at stator (22) and a rotor (24) rotatably mounted in the stator (22), the stator comprising at least two stator windings (34), **characterized in that** the stator windings (34) are wound in a self-supporting or coil carrier-free manner, and **in that** after the winding of the at least two stator windings (34), a tubular protective sleeve (38) made of a ceramic is inserted into the at least two stator windings (34) wound in a tubular shape.

17. Method in accordance with claim 16, **characterized in that** after insertion of the protective sleeve (38), the at least two stator windings (34) are cast with an embedding or casting compound (44) to form a winding layer (46) containing the at least two stator windings (34).

## Revendications

1. Moteur électrique (16) pour applications chirurgicales, comportant un stator (22) et un rotor (24) monté en rotation dans le stator (22), le stator (22) comprenant au moins deux enroulements de stator (34), le stator (22) comprenant également une gaine de protection (38), et lesdits au moins deux enroulements de stator (34) entourant la gaine de protection (38), **caractérisé en ce que** lesdits au moins deux enroulements de stator (34) sont bobinés en porte-à-faux, et **en ce que** la gaine de protection (38) est fabriquée en une céramique.

2. Moteur pour applications chirurgicales selon la revendication 1, **caractérisé en ce que** la gaine de protection (38) est fabriquée en un matériau pouvant être stérilisé à la vapeur.

3. Moteur pour applications chirurgicales selon la revendication 1 ou la revendication 2, **caractérisé en ce qu'**entre le stator (22) et le rotor (24) est formé un entrefer (60), et **en ce que** la gaine de protection (38) est directement adjacente à l'entrefer (60).

4. Moteur pour applications chirurgicales selon l'une des revendications précédentes, **caractérisé en ce que** la gaine de protection (38) présente une épaisseur d'au maximum 0,5 mm.

5. Moteur pour applications chirurgicales selon l'une des revendications précédentes, **caractérisé en ce que** la gaine de protection (38) est fabriquée en un matériau isolant sur le plan électrique.

6. Moteur pour applications chirurgicales selon l'une des revendications précédentes, **caractérisé en ce que** la céramique est une céramique oxydée ou un oxyde technologique.

7. Moteur pour applications chirurgicales selon la revendication 6, **caractérisé en ce que** la céramique oxydée est et/ou renferme de l'oxyde d'aluminium (Al₂O₃), de l'oxyde de zirconium (ZrO₂), de l'oxyde de titane (TiO₂), de l'oxyde de magnésium (MgO), de l'oxyde de zinc (ZnO), du titanate d'aluminium (Al₂O₃ + TiO₂) et/ou du titanate de baryum (BaO + TiO₂).

8. Moteur pour applications chirurgicales selon l'une des revendications précédentes, **caractérisé en ce que** lesdits au moins deux enroulements de stator (34) sont noyés dans une masse d'enrobage ou de scellage (44), et **en ce que** lesdits au moins deux enroulements de stator (34) et la masse d'enrobage ou de scellage (44) forment une couche d'enroulement (46).

9. Moteur pour applications chirurgicales selon la revendication 8, **caractérisé en ce que** la masse d'enrobage ou de scellage (44) est isolante sur le plan électrique.

10. Moteur pour applications chirurgicales selon la revendication 8 ou la revendication 9, **caractérisé en ce que** la masse d'enrobage ou de scellage (44) est conductrice sur le plan magnétique.

11. Moteur pour applications chirurgicales selon l'une des revendications 8 à 10, **caractérisé en ce que** la masse d'enrobage ou de scellage (44) est réalisée dans le même matériau que la gaine de protection (38).

12. Moteur pour applications chirurgicales selon l'une des revendications 8 à 11, **caractérisé en ce que** la masse d'enrobage ou de scellage (44) est dopée par couches.

13. Moteur pour applications chirurgicales selon l'une des revendications 8 à 12, **caractérisé en ce que** la masse d'enrobage ou de scellage (44) est dopée avec des particules (52) magnétiques et/ou pouvant être magnétisées.

14. Moteur pour applications chirurgicales selon l'une des revendications précédentes, **caractérisé en ce que** lesdits au moins deux enroulements de stator (34) sont agencés entre la gaine de protection (38) et une couche d'isolation (54) isolante sur le plan électrique.

15. Machine (10) pour applications chirurgicales, comprenant un entraînement (16) destiné à mouvoir un outil de travail, qui peut être couplé directement ou indirectement avec l'entraînement (16), l'entraînement (16) étant un moteur électrique (16) pour applications chirurgicales selon l'une des revendications précédentes.

16. Procédé pour la fabrication d'un moteur électrique (16) pour applications chirurgicales, comportant un stator (22) et un rotor (24) monté en rotation dans le stator (22), le stator (22) comprenant au moins deux enroulements de stator (34), **caractérisé en ce que** les enroulements de stator (34) sont bobinés en porte-à-faux ou sans support d'enroulement, et **en ce qu'**après le bobinage desdits au moins deux enroulements de stator (34), on insère une gaine de protection (38) de forme tubulaire, fabriquée en une céramique, dans lesdits au moins deux enroulements de stator (34) bobinés sous forme tubulaire.

17. Procédé selon la revendication 16, **caractérisé en ce que** lesdits au moins deux enroulements de stator (34), après l'insertion de la gaine de protection (38), sont noyés dans une masse d'enrobage ou de scellage (44), pour former une couche d'enroulement (46) renfermant lesdits au moins deux enroulements de stator (34).
